# EUROPEAN PATENT APPLICATION

(11) **EP 3 733 872 A1**
(43) Date of publication of application: **04.11.2020**
(21) Application number: 18893817.9
(22) Date of filing: 28.12.2018
(51) Int. Cl.: C12Q 1/6844, C12Q 1/6869, C40B 50/06

(54) **METHOD FOR QUICKLY HOMOGENIZING CIRCULAR DNA SAMPLES**

(30) Priority: 28.12.2017 CN 201711462509
(71) Applicant: Nanjingjinsirui Science & Technology Biology Corp., Nanjing, Jiangsu 211100 (CN)
(72) Inventor: FAN, Long, Nanjing, Jiangsu 211100 (CN); LIU, Jiadong, Nanjing, Jiangsu 211100 (CN); JIANG, Haojun, Nanjing, Jiangsu 211100 (CN); ZHOU, Jingbo, Nanjing, Jiangsu 211100 (CN); WU, Cheng-Hsien, Nanjing, Jiangsu 211100 (CN)
(74) Representative: Pharma Patents International AG
(86) International application number: PCT/CN2018/124720
(87) International publication number: WO 2019/129184

(57) **Abstract**

The present application provides a method for quickly homogenizing circular DNA samples, comprising performing rolling circle amplification on circular DNAs in the samples, so that the concentration of the circular DNAs in the samples are homogenized.

## Description

### Technical Field

The present invention relates to the field of biological engineering, and in particular, to a homogenization method suitable for samples comprising circular DNAs. The method is specifically a method for quickly homogenizing circular DNA samples by a rolling circle replication.

### Background Art

The experimental process of next-generation sequencing (NGS) mainly involves three steps: sample template preparation, sequencing library construction, and on-line sequencing. Among these steps, the sequencing library construction needs to control the initial sample amount of different detection samples, so that the concentrations of different samples are homogenized to facilitate downstream experimental operations. The conventional homogenization method (i.e., quantification - calculation - pipetting) mainly estimates the amount of DNAs contained through the absorbance value, so as to pipette the same amount of samples. However, the method using the absorbance value or fluorescence quantification will be taffected by other proteins, other types of nucleic acids or impurities with similar absorption of specific spectra, and the fluorescence quantification has the disadvantages of high cost, tedious and time-consuming operation, etc. Specifically, the operation time of homogenizing 10,000 samples is as follows: (1) for the calculation step, it takes about 100 hours to input the concentration of each sample and calculate the specific pipetted sample amount; and (2) for the process of adjusting a pipette and pipetting the corresponding calculated amount of samples separately from each sample to achieve homogenization between samples, it takes 100 hours. Therefore, according to the existing technical process, the entire homogenization process requires a manual operation time of 200 hours.

The rolling circle amplification (RCA) technology can be used to amplify circular DNA templates, such as plasmids and phage DNAs. This technology is a nucleic acid amplification technology established on the basis of the method of rolling circle replication of circular DNA molecules of pathogenic microorganisms in nature, and it is a DNA amplification technology that occurs at a constant temperature (Lizardi *et al*., 1998).

An RCA system comprises: (1) phage phi29 DNA polymerase; (2) a circular template (currently the RCA amplification template may also be linear); and (3) primers, which generally need to be resistant to endonuclease. Among these, the phage phi29 DNA polymerase is very important It has high strand displacement activity, and can perform 70 kb strand displacement DNA synthesis without template separation. Moreover, it has a stable performance and a high continuous synthesis capacity, and can efficiently catalyze DNA synthesis for several hours at a synthesis speed of about 50 bp/s. In addition, the phi29 DNA polymerase has strong error correction ability, and the error rate is comparable to that of pfu enzyme, and significantly lower than that of Taq DNA polymerase. RCA is an isothermal signal amplification method with a linear amplification factor of 10⁵ and an exponential amplification capacity of greater than 10⁹. The RCA technology is a trace molecular detection method that can be used for the detection and research of extremely trace biological macromolecules and biomarkers. At present, this method has been successfully used for the amplification of linear double-stranded DNAs and even whole genomic DNAs (Esteban *et al.*, 1993; Qian *et al.*, 2003; Simison *et al.*, 2006). Therefore, the RCA technology, with its unique advantages, provides the possibility of sample homogenization before the NGS library construction.

The present invention optimizes the rolling circle amplification (RCA) technology, and uses it to amplify circular DNA templates, such as plasmids and phage DNAs, to homogenize different concentrations of circular DNA samples, so that the coefficient of variation of the concentrations of DNA templates of different samples is lower than 0.1, and the manual operation time is less than 5 hours.

### Summary of the Invention

An objective of the present invention is to solve the problems of high cost and a long consuming time for homogenizing large quantities of samples in the preparation of DNA libraries, especially in the preparation of sequencing libraries for NGS.

In order to solve this problem, in one aspect, the present application provides a method for homogenizing circular DNA samples based on a rapid rolling circle amplification, comprising: performing rolling circle amplification on each sample, so that the concentrations of the circular DNAs in the samples are homogenized.

According to some embodiments, the homogenization method comprises a step of performing denaturing treatment on each sample before performing the rolling circle amplification. In the context of the present application, "denaturing" is understood in accordance with the ordinary meaning of nucleic acid denaturation in the art According to some further embodiments, the denaturing treatment comprises treating the samples at an elevated temperature until the denaturation is completed, and then lowering the temperature of the samples. According to some more specific embodiments, the denaturing treatment is performed by heating the samples to a temperature of about 95ºC for 3 minutes. According to some more specific embodiments, the samples are cooled to 4ºC after heating, and kept According to some further embodiments, random primers for the rolling circle amplification are added to a reaction system of the denaturing treatment. In some specific embodiments, the random primers are 7 bp in length. Without wishing to be bound by any theory, the high temperature can ensure the sufficient release of the initial template DNAs, and the cooling process can allow the random primer(s) and the circular DNA template to be annealed. By adding random primers before the denaturation process, compared with addition of random primers after the denaturation process, the effect of homogenizing circular DNAs by rolling circle amplification can be further improved.

In some embodiments, the reaction system of the denaturing treatment comprises EDTA. In some preferred embodiments, the concentration of EDTA in the reaction system of the denaturing treatment is about 0.05 mM. In some more specific embodiments, the denaturing treatment preferably comprises the steps of: to 2 µl of an amplification template sample, adding 5 µl of a denaturing buffer (10 mM Tris-HCl, 0.1 mM EDTA, pH 8.0, 25°C), and adding 3 µl of random primers (100-200 uM), mixing well, treating at 95ºC for 3 minutes to complete the denaturation, and cooling to 4ºC.

In some embodiments, the reaction system of the rolling circle amplification comprises phi29 DNA polymerase. In some further embodiments, the rolling circle amplification comprises an isothermal amplification at a temperature of 30ºC for 3 hours. In some further embodiments, the reaction system of the rolling circle amplification comprises bovine serum albumin (BSA). In some further embodiments, the concentration of bovine serum albumin (BSA) is about 0.1 mg/mL. In some embodiments, the reaction system of the rolling circle amplification comprises KCl. In some further embodiments, the concentration of KCl in the reaction system of the rolling circle amplification is about 75 mM.

According to a specific embodiment of the method of the present invention, the rolling circle amplification preferably comprises the steps of: to the product which has been subjected to the step of denaturing treatment, adding 2 µl of 10x amplification buffer (500 mM Tris-HCl, 50 mM MgCl₂, 750 mM KCl, 50 mM DTT, pH 8.2, 25°C), 0.2 µl of BSA (10-50 mg/ml), 2.4 µl of dNTPs (2.5-10 mM), 3 µl of phi29 DNA polymerase (5-10 U/µl], and 2.4 µl of ddH₂O, made up to 20 µl of the final total reaction system, mixing well, amplifying at 30ºC for 4 hours, treating at 65ºC for 10 minutes, and cooling to 4ºC.

In a preferred embodiment, the homogenization method of the present invention further comprises treating the amplification product with an endonuclease after the completion of the rolling circle replication and amplification. In a further embodiment, the endonuclease is a BamH1-HF endonuclease.

In a preferred embodiment, the homogenization method of the present invention comprises neither a step of quantifying an initial amount of the circular DNAs in the samples, nor a step of calculating a sample amount to be pipetted based on the measured amount of the circular DNAs in the samples.

The sample applicable to the present invention may be any biological sample comprising or consisting substantially of circular DNAs, for example, without limitation, bacterial solutions, colonies, cell debris, plasmids, M13 phage, and any other circular DNA samples. The sample may or may not be purified before being applied to the method of the present invention.

The method of the present invention can be used in any application where it is desired to homogenize a plurality of samples containing circular DNAs, including, but not limited to, the preparation of DNA libraries, especially DNA libraries for next-generation sequencing (NGS).

Therefore, in one aspect, the present application provides a method for preparing a DNA library from a plurality of samples containing circular DNAs, comprising treating each sample using the homogenization method of the present invention. In a preferred embodiment, the method for preparing a library comprises pipetting the same amount of samples to prepare a library after treating a plurality of samples using the homogenization method of the present invention.

In another aspect, the present application provides a next-generation sequencing method, comprising treating a plurality of samples to be sequenced using the homogenization method of the present invention to prepare a sequencing library, and performing a next-generation sequencing on the sequencing library. The sequencing method can be used on any next-generation sequencing platform, including, but not limited to, Roche 454 platform, Illumina Solexa platform, ABI SOliD platform, etc.

### Beneficial Effects

The rolling circle amplification method used in the present invention is generated by optimizing based on the commercial reagents and the rolling circle amplification method reported in reference documents. This rolling circle amplification method optimizes the existing rolling ring amplification system, reagents and method flow as a whole; and effectively solves the problem of circular DNA homogenization, especially the circular DNA homogenization before the large-scale NGS library construction, on the premise of ensuring that the library construction effect and sequencing effect are comparable to the effects in the case of a traditional DNA homogenization. Conventional DNA homogenization of 10,000 samples requires a process of quantification - calculation - pipetting, and it will take 200 hours to perform the above operation on a large-scale sample. Since the method of the present invention does not require the operation of quantification - calculation - pipetting, an automatic pipetting platform can be used to complete the pretreatment - homogenization process of 10,000 NGS library samples within 4.5 hours, which greatly shortens the time of sample pretreatment In general, the method for homogenizing the NGS library samples based on rolling circle amplification provided by the present invention solves the shortcoming of time consuming and tedious operation in the sample homogenization at an early stage of the large-scale NGS library construction, and its unique design can realize the early-stage homogenization of NGS library samples quickly and easily.

### Brief Description of the Drawings:

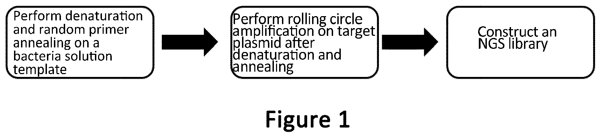

Figure 1 shows a flow chart of homogenization of circular DNAs of the present invention.

### Detailed Description of Embodiments:

In order to further understand the method described in the present invention, the present invention will be further described below with reference to the accompanying drawings and examples.

### Example 1:

This example compares the homogenization effect (i.e. the coefficient of variation quantified by Qubit) of the concentrations of DNA templates prepared by three different rolling circle amplification methods within the same time:
A. Related procedures for NEB phi29 DNA polymerase reagent (Cat# M0269S, this reagent provides some supporting reagents and experimental protocols for RCA reactions) (hereinafter referred to as "NEB reagent (phi29 DNA polymerase)")
B. RCA experimental protocol cited from literature (Frank B. Dean., John R. Nelson., Theresa L. Giesler., and Roger S. Lasken. 2001. Rapid Amplification of Plasmid and Phage DNA Using Phi29 DNA Polymerase and Multiply-Primed Rolling Circle Amplification. *Genome Research.* 1095-1099) (hereinafter referred to as the "methods in the literature")
C. The homogenization method provided by the present application (hereinafter referred to as "the present application")

The test samples were 16 bacterial solutions used in actual production which were transformed with plasmids. The OD600 value of each bacterial solution was measured with a spectrophotometer, and the results are shown in Table 1.

Three different sequencing template preparation processes were used to prepare sequencing templates, as described below:

### 1) Step 1 (denaturation process): performing denaturation and random primer annealing on a bacterial solution template

The experimental samples were bacterial solutions transformed with plasmids as described above. The system configurations of denaturation reactions for three different sequencing template preparation methods are listed in the following table respectively:

| **NEB reagent (phi29 DNA polymerase)** | |
|---|---|
| **Component** | **Volume (µl)** |
| Bacterial solution | 1 |
| 10x reaction buffer (500 mM Tris-HCl, 100 mM MgCl₂, 100 mM (NH₄)₂SO₄, 40 mM DTT, pH 7.5, 25°C) | 1 |
| 7 bp random primer (100 uM) | 2.5 |
| ddH₂O | 4.3 |
| Total volume | 8.8 |

| **Methods in the literature** | |
|---|---|
| Component | Volume (µl) |
| Bacterial solution | 2 |
| TE buffer (10 mM Tris-HCl, 1 mM EDTA, pH 8.0, 25°C) | 8 |
| Total volume | 10 |

| **The present application** | |
|---|---|
| Component | Volume (µl) |
| Bacterial solution | 2 |
| Denaturing buffer (10 mM Tris-HCl, 0.1 mM EDTA, pH 8.0, 25°C) | 5 |
| 7 bp random primer (175 uM) | 3 |
| Total volume | 10 |

Three different sequencing template preparation methods used the same experimental operation flow of denaturation reaction, as shown in the following table:

| Temperature | Time (min) |
|---|---|
| 95°C | 3 |
| 4°C | Keeping |

### 2) Step 2 (amplification process): performing isothermal rolling circle amplification on target plasmid after denaturation and annealing

Using the reaction product from Step 1 as an experimental sample, the isothermal rolling circle amplification was performed according to three different sequencing template preparation methods. The system configuration of the amplification reaction in each method is shown in the following table:

| NEB reagent (phi29 DNA polymerase) | |
|---|---|
| Component | Volume (µl) |
| Reaction product from Step 1 | 8.8 |
| dNTP (10 mM) | 0.5 |
| BSA (10 mg/mL) | 0.2 |
| Phi29 DNA polymerase (10 U/µl] | 0.5 |
| Total volume | 10 |

| Methods in the literature | |
|---|---|
| Component | Volume (µl) |
| Reaction product from Step 1 | 10 |
| 10x amplification buffer (500 mM Tris-HCl, 50 mM MgCl₂, 750 mM KCl, 1 mM DTT, pH 8.2, 25°C) | 2 |
| 7 bp random primer (100 uM) | 1 |
| dNTP (2.5 mM) | 0.8 |
| Phi29 DNA polymerase (10 U/µl] | 0.5 |
| Yeast pyrophosphatase (0.1 U/µl] | 0.3 |
| ddH₂O | 5.4 |
| Total volume | 20 |

| The present application | |
|---|---|
| Component | Volume (µl) |
| Reaction product from Step 1 | 10 |
| 10x reaction buffer (500 mM Tris-HCl, 50 mM MgCl₂, 750 mM KCl, 50 mM DTT, pH 8.2, 25°C) | 2 |
| BSA (10 mg/mL) | 0.2 |
| dNTP (3 mM) | 2.4 |
| Phi29 DNA polymerase (5 U/µl] | 3 |
| ddH₂O | 2.4 |
| Total volume | 20 |

Three different sequencing template preparation methods used the same experimental operation flow of isothermal amplification, as shown in the following table:

| Temperature | Time |
|---|---|
| 30°C | 4 hours |
| 65°C | 10 minutes |
| 4°C | Keeping |

### 3) RCA product treatment and quantification

The RCA product has a high-order structure; therefore, in this example, all the RCA products were enzymatically digested with BamH1-HF to make the quantification more accurate. The NEB reagent (phi29 DNA polymerase) was configured by a digestion reaction system, and the methods in the literature and the method provided in the present application were configured by another enzymatic digestion reaction system, as described in the following table:

| NEB reagent (phi29 DNA polymerase) | |
|---|---|
| Component | Volume (µl) |
| 10x Cut Smart Buffer (Cat# B7203S, 500 mM potassium acetate 200 mM Tris-acetic acid 100 mM magnesium acetate 1,000 ug/ml BSA pH 7.9, 25°C) | 3 |
| RCA product | 10 |
| BamH1-HF (2 U/µl, Cat# R3136S) | 1 |
| ddH₂O | 16 |
| Total volume | 30 |

| Methods in the literature/The present application | |
|---|---|
| Component | Volume (µl) |
| 10x Cut Smart Buffer (Cat# B7203S, 500 mM potassium acetate 200 mM Tris-acetic acid 100 mM magnesium acetate 1,000 ug/ml BSA pH 7.9, 25°C) | 3 |
| RCA product | 20 |
| BamH1-HF (2 U/µl, Cat# R3136S) | 1 |
| ddH₂O | 6 |
| Total volume | 30 |

The experimental operation flow of digestion reaction in three different sequencing template preparation methods is consistent:

| Temperature | Time |
|---|---|
| 37°C | 1 hour |
| 65°C | 20 minutes |
| 4°C | Keeping |

4 µl of each sample after the above reaction was quantified using a Qubit™ fluorescence quantifier (Cat# Q33216, Thermo Fisher). The results are shown in Table 2. Based on the raw data in Tables 1 and 2, the coefficients of variation (ratio of standard deviation to mean) of the OD600 measurement of each initial bacterial solution, and of the Qubit values of the DNA templates respectively prepared by using the NEB phi29 DNA polymerase kit, the method in the literature, and the method of the present application in Table 2 were calculated, respectively. The results are shown in Table 3.

### Results

In this example, the use of the method provided in the present application successfully achieves the homogenization of circular DNA samples. Meanwhile, we compared the differences among the method of NEB reagent product and the method reported in the literature and the method of the present application. The coefficient of variation of the OD600 measurement (see Table 1) before treatment on an initial bacterial solution was 0.408 (see Table 3); the coefficient of variation of the Qubit value (see Table 2 for details) of the DNA template obtained by treating with NEB reagent was 0.174 (see Table 3); and the coefficient of variation of the Qubit value (see Table 2) of the DNA template obtained by treating with the RCA method reported in the literature was 0.12 (see Table 3). The coefficient of variation of the Qubit value (see Table 2) of the DNA template obtained by the method of the present application was 0.086 (see Table 3). It can be seen that the coefficients of variation of the concentrations (see Table 2) of the DNA templates obtained after three treatments are all lower than the coefficient of variation of the OD value of the initial bacterial solution, and the effect of homogenizing the content of circular DNAs in the samples is achieved. In particular, the coefficient of variation of the sample obtained by the method of the present application is significantly lower than that of the sample obtained by the NEB reagent treatment and the method in the literature, indicating that the method provided by the present application further improves the homogenization effect of DNA samples.

**Table 1: OD600 measurements of the bacterial solution samples of Example 1**

| Bacterial Solution | OD600 | Bacterial Solution | OD600 |
|---|---|---|---|
| Bacterial solution 1 | 0.269 | Bacterial solution 9 | 0.308 |
| Bacterial solution 2 | 0.38 | Bacterial solution 10 | 0.286 |
| Bacterial solution 3 | 0.264 | Bacterial solution 11 | 0.291 |
| Bacterial solution 4 | 0.331 | Bacterial solution 12 | 0.305 |
| Bacterial solution 5 | 0.308 | Bacterial solution 13 | 0.658 |
| Bacterial solution 6 | 0.277 | Bacterial solution 14 | 0.255 |
| Bacterial solution 7 | 0.772 | Bacterial solution 15 | 0.354 |
| Bacterial solution 8 | 0.305 | Bacterial solution 16 | 0.341 |

**Table 2: Qubit measurements of the amplified products obtained in each experimental method of Example 1**

| Bacterial solution | RCA product concentration (ng/µL, after enzymatic digestion) | | |
|---|---|---|---|
| | NEB phi29 DNA polymerase reagent (Cat# M0269S) | Methods in the literature | The present invention |
| Bacterial solution 1 | 39.4 | 6.1 | 29 |
| Bacterial solution 2 | 39 | 7 | 35.5 |
| Bacterial solution 3 | 44.2 | 5.7 | 29.7 |
| Bacterial solution 4 | 42.4 | 6.75 | 32.7 |
| Bacterial solution 5 | 42.8 | 6.75 | 33.3 |
| Bacterial solution 6 | 43 | 6.6 | 31.2 |
| Bacterial solution 7 | 39.4 | 6.2 | 30.8 |
| Bacterial solution 8 | 38.8 | 7.3 | 34.4 |
| Bacterial solution 9 | 18.6 | 7.55 | 31.3 |
| Bacterial solution 10 | 33.6 | 6.6 | 33 |
| Bacterial solution 11 | 47.4 | 5.7 | 31.8 |
| Bacterial solution 12 | 39.4 | 6.35 | 32.9 |
| Bacterial solution 13 | 47 | 5.4 | 26.9 |
| Bacterial solution 14 | 48.1 | 5.15 | 26.6 |
| Bacterial solution 15 | 45 | 7.5 | 35.8 |
| Bacterial solution 16 | 38.2 | 7.7 | 33.8 |

**Table 3: Comparison table for coefficients of variation (CV) of the sample concentrations of Example 1.**

| Type of measurement data | Coefficient of variation (CV) |
|---|---|
| OD600 value of the initial bacterial solution | 0.408 |
| Qubit value of the DNA template prepared by homogenization using the NEB phi29 DNA polymerase kit | 0.174 |
| Qubit value of the DNA template prepared by homogenization using the method in the literature | 0.12 |
| Qubit value of the DNA template prepared by homogenization using the method of the present invention | 0.086 |

In addition, the present invention is easy to use in connection with an automatic pipetting platform. Taking Example 1 as an example, after combining the present invention with an automatic pipetting platform, the time taken to homogenize 10,000 samples using the method of the present invention can be controlled within 5 hours; while the time taken by the conventional homogenization method (i.e. quantification - calculation - pipetting) increases linearly with the increase of the sample amount, and for the homogenization of 10,000 samples to be treated, it takes up to about 200 hours.

## Claims

1. A method for homogenizing a plurality of samples comprising circular DNAs, comprising: performing rolling circle amplification on circular DNAs in the samples, so that the concentrations of the circular DNAs in the samples are homogenized.

2. The method of claim 1, wherein the method further comprises performing denaturing treatment on each sample before performing the rolling circle amplification.

3. The method of claim 2, wherein the samples are mixed with random primers for the rolling circle replication before starting the denaturing treatment

4. The method of claim 2 or 3, wherein the denaturing treatment comprises heating the samples to 95ºC and keeping for 3 minutes, and then cooling to 4ºC and keeping.

5. The method of any one of claims 2 to 4, wherein a system of the denaturing treatment comprises EDTA, preferably about 0.05 mM EDTA.

6. The method of any one of claims 1 to 5, wherein a reaction system of the rolling circle amplification comprises bovine serum albumin, preferably about 0.1 mg/mL bovine serum albumin.

7. The method of any one of claims 1 to 6, wherein the reaction system of the rolling circle amplification comprises KCl, preferably about 75 mM KCl.

8. The method of any one of claims 1 to 7, wherein the method comprises neither a step of quantifying an initial amount of the circular DNAs in the samples, nor a step of calculating a sample amount to be pipetted based on the measured amount of the circular DNAs in the samples.

9. A method for preparing a DNA library from a plurality of samples comprising circular DNAs, comprising treating the samples using the method of any one of claims 1 to 8.

10. A next-generation sequencing method, comprising: treating a plurality of samples to be sequenced using the method of any one of claims 1 to 8 to prepare a sequencing library, and performing a next-generation sequencing on the sequencing library.
